(19) Europäisches Patentamt
European Patent Office
Office européen des brevets

(11) Veröffentlichungsnummer: **0 000 533**
**B1**

(12) # EUROPÄISCHE PATENTSCHRIFT

(45) Veröffentlichungstag der Patentschrift: **05.08.81**

(51) Int. Cl.[3]: **C 07 D 457/04**

(21) Anmeldenummer: **78100419.7**

(22) Anmeldetag: **18.07.78**

(54) **N-Substituierte 9,10-Dihydrolysergsäureester sowie ein Verfahren zu deren Herstellung.**

(30) Priorität: **21.07.77 YU 1819/77**

(43) Veröffentlichungstag der Anmeldung:
**07.02.79 Patentblatt 79/3**

(45) Bekanntmachung des Hinweises auf die Patenterteilung:
**05.08.81 Patentblatt 81/31**

(84) Benannte Vertragsstaaten:
**BE CH DE FR GB SE**

(56) Entgegenhaltungen:
**FR - A - 1 175 276**

(73) Patentinhaber: **LEK, tovarna farmacevtskih in kemicnih izdelkov, n.sol.o.**
**Celovška 135**
**YU-61000 Ljubljana (YU)**

(72) Erfinder: **Rucman, Rudolf**
**Ptujska 25**
**YU-61000Ljubljana (YU)**

(74) Vertreter: **Deufel, Paul et al,**
**Patentanwälte MÜLLER-BORÉ-DEUFEL SCHÖN-HERTEL Siebertstrasse 4**
**D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 533 B1

Courier Press, Leamington Spa, England.

N-Substituierte, 9,10-Dihydrolysergsäureester sowie ein Verfahren zu deren Herstellung

Die Erfindung betrifft N-substituierte 9,10-Dihydrolysergsäureester sowie ein Verfahren zu deren Herstellung.

Die erfindungsgemäßen N-substituierten 9,10-Dihydrolysergsäureester der allgemeinen Formel I

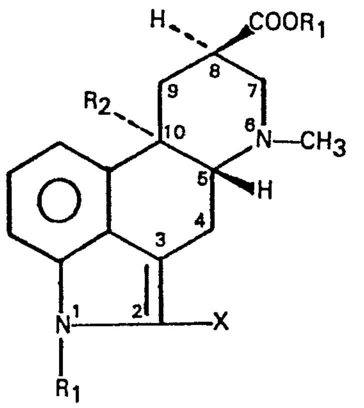

(I)

worin

$R_1$ einen Alkylrest mit 1—5 C-Atomen, einen Alkenylrest mit 2—5 C-Atomen oder einen Cycloalkylrest mit 3—5 C-Atomen,

$R_2$ ein Wasserstoff atom oder einen Alkoxyrest mit 1—3 C-Atomen und

X ein Wasserstoff- oder Halogen atom bedeuten, wobei die Verbindungen, worin $R_1$ einen Methyl- oder Äthylrest und $R_2$ und X jeweils ein Wasserstoff atom bedeuten, sowie die Verbindung, worin $R_1$ einen Methylrest, $R_2$ einen Methoxyrest und X ein Wasserstoff atom bedeuten, ausgenommen sind,

sind wichtige Zwischenverbindungen für die Synthese von therapeutisch hochwirksamen Verbindungen.

Das bisher bekannte Verfahren der N-Alkylierung von Lysergsäure derivaten oder anderen Verbindungen dieser Basisstruktur beruht auf der Metallisierung des Indolstickstoff atoms mit Kalium oder Lithium in flüssigem Ammoniak bei —50°C und anschliessender Einführung der Alkylgruppe. Gemäß diesem Verfahren wird die Alkylgruppe nur in die 1-Stellung eingeführt. Da die Reaktion nicht besonders selektiv ist, wird sie, insbesondere beim notwendigen Überschuss von Alkylhalogenid, auch durch die Substitution des aktivierten Wasserstoff atoms am Chiralitätszentrum 8 mit einer Alkylgruppe begleitet, so dass die Ausbeute an N-Alkylverbindung geringer wird vgl. F. Troxler und A. Hofmann, Helv. chim. Acta *40*, (1957), 1721]. Die Arbeit unter den angegebenen Reaktionsbedingungen ist aufwendig, es sind wasserfreie Lösungsmittel erforderlich und under ungünstigen Bedingungen kann es auch zu Explosionen kommen. Das Reaktionsprodukt muss durch Säulenchromatographie gereinigt werden.

Das erfindungsgemässe Verfahren zur Herstellung von N-alkylierten 9,10-Dihydrolysergsäureestern der allgemeinen Formel I ist dadurch gekennzeichnet, dass die gegebenenfalls veresterte 9,10-Dihydrolysergsäure der allgemeinen Formel II

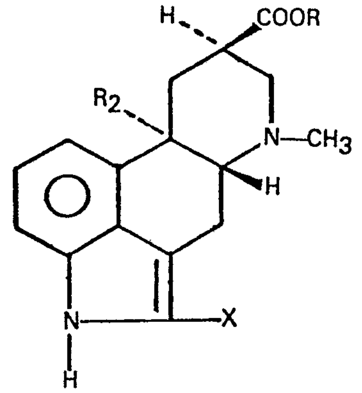

(II)

worin

R ein Wasserstoff atom oder einen in alkalischem Medium hydrolisierbaren organischen Rest bedeutet, und

X und $R_2$ die angegebene Bedeutung haben,

mit einer Verbindung der allgemeinen Formel III

$$R_1Y \qquad \text{(III)}$$

worin

$R_1$ die angegebene Bedeutung hat, und

Y das Äquivalent eines Halogen- oder Sulfat ions bedeutet, in Anwesenheit eines Katalysators zur Phasenübertragung der allgemeinen Formel IV

$$[Z_4Q] \quad A \qquad \text{(IV)}$$

in der

Z jeweils eine gleiche oder verschiedene Alkyl-, Cycloalkyl-, Aryl-, Arylalkyl- oder Alkylarylgruppe mit 1 bis 16 C-Atomen, Q ein quaternäres Stickstoff- oder ein Phosphoratom und A das Äquivalent eines Anions bedeutet, eines mit Wasser nicht mischbaren inerten organischen Lösungsmittels und einer alkalischen wässrigen Phase umgesetzt wird.

Nach dem erfindungsgemässen Verfahren wird gleichzeitig in die 1-Stellung der Verbindung der allgemeinen Formel II die Alkylgruppe eingeführt und die Gruppe in der 8-Stellung wird durch dieselbe Alkylgruppe, die in die 1-Stellung eingeführt wurde, verestert, bzw. umgeestert.

In der Verbindungn der allgemeinen Formel II bedeutet R ein Wasserstoff atom oder einen in alkalischem Medium hydrolisierbaren organischen Rest. Deswegen können für das erfindungsgemässe Verfahren auch die jenigen 9,10-Dihydrolysergsäureester verwendet werden, bei denen R irgend einen organischen Rest bedeutet, der höher molekular als die eingeführte Gruppe R ist. Dieser organische Rest R wird durch die intermediäre alkalische Hydrolyse durch eine niedrigere Gruppe $R_1$ ersetzt.

Die Verbindung der allgemeinen Formeln II und III sind bekannt und beschrieben oder können durch an sich bekannte Verfahren hergestellt werden.

Als Katalysator zur Phasenübertragung können beispielsweise Tetrabutylammoniumbromid, Tetrabutylammoniumhydrogensulfat, Triäthylbenzylammoniumchlorid, Tricaprylmethylammoniumchlorid oder Tetrabutylphosphoniumbromid eingesetzt werden.

Der Katalysator zur Phasenübertragung wird in einer Menge von 0,1 bis 3 Mol auf 1 Mol 9,10-Dihydrolysergsäure eingesetzt. Die Menge des Katalysators in diesen Grenzen beeinflusst sehr die Reaktionsgeschwindigkeit. Da 9,10-Dihydrolysergsäure und ihre Derivate sehr empfindliche Verbindungen sind, ist ein schneller Reaktionsverlauf erwünscht. Deswegen ist die Verwendung des Katalysators in einer Menge vorteilhaft, die nahe an der angeführten oberen Grenze liegt.

Das mit Wasser nicht mischbare inerte organische Lösungsmittel kann z.B. Benzol, Toluol, Xylol oder ein gesättigter Kohlenwasserstoff, wie Pentan, Hexan, Heptan oder Cyclohexan, sein.

Die alkalische wässerige Phase ist eine 20—50%ige wässerige Alkalihydroxydlösung, z.B. Natronlauge.

Das erfindungsgemässe Verfahren wird bei Raumtemperatur oder mässig erhöhter Temperatur durchgeführt.

Die Substitution der 9,10-Dihydrolysergsäurederivate in der 8-Stellung verläuft schneller als in der 1-Stellung. Deswegen können am Anfang der Reaktion aus dem Reaktionsgemisch der Ester und der N-substituierte Ester isoliert werden. Durch die Weiterführung der Reaktion wird die Konzentration des Esters vermindert, bis sie am Ende der Reaktion vollkommen verschwindet und nur der N-substituierte Ester erhalten wird. Deswegen kann bei der erfindungsgemäßen Einführung einer Alkyl-, Alkenyl- oder Cycloalkylgruppe in einen 9,10-Dihydrolysergsäurealkyl-, -alkenyl oder -cycloalkylester in hoher Ausbeute ein 1-(Alkyl oder Alkenyl oder Cycloalkyl) - 9,10 - dihydrolysergsäurealkyl-, -alkenyl- oder -cycloalkylester erhalten werden.

Die Vorteile des erfindungsgemässen Verfahrens gegenüber dem bekannten Verfahren sind gute Selektivität, einfacher und schneller Reaktionsverlauf, wobei die Reaktion leicht kontrolliert werden kann, Verwendung von üblichen Lösungsmitteln, aus welchen das Wasser nicht entfernt zu werden braucht, vor allem jedoch die Reaktionsbedingungen, die eine gefahrlose Arbeit ermöglichen.

Die erfindungsgemässen Verbindungen sind wichtige Zwischenverbindungen zur Synthese von therapeutisch hochwirksamen Verbindungen mit N-substituierter Gruppe in 1-Stellung. Die weiteren Umsetzungen der Synthese verlaufen vor allem in Richtung Reduktion der Estergruppe in der 8-Stellung zu primären Alkoholgruppe, in die dann ein geeigneter Säurerest, z.B. der 5-Bromnicotinsäurerest, eingeführt wird. Deswegen ist die gleichzeitige Einführung der Gruppe $R_1$ in die 1-Stellung und die Veresterung der Carboxygruppe in der 8-Stellung, die dadurch für die Reduktion in die Alkoholgruppe zugänglicher wird, sehr vorteilhaft.

Beispiel 1

2,7 g (10 mMol) 9,10-Dihydrolysergsäure und 7 g (20 mMol) Tetrabutylammoniumhydrogensulfat werden in 200 ml 45%iger Natronlauge suspendiert. Eine Lösung von 4,2 g (30 mMol) Methyljodid in 300 ml Benzol wird zugegeben und 1 Stunde intensiv gerührt. Die Organische Phase wird dann abgetrennt und die wässerige Phase in gleicher Weise noch 2 mal mit je 150 ml Benzol, das 2,1 g (15 mMol) Methyljodid enthält, extrahiert. Die Benzolextrakte werden vereinigt, mit Wasser bis zur neutralen Reaktion gewaschen und das Lösungsmittel z.b. gedampft. Es werden 2,62 g bzw. 88% d.Th. reinen, kristallinen 1-Methyl-9,10-dihydrolysergsäuremethylesters mit einem Schmp. von 116—119°C und einer spezifischen Drehung von $[\alpha]_D^{20} = -61,1°$ (c = 0,5; Chloroform) erhalten.

Beispiel 2

In ein aus 200 ml 50%iger Natronlauge 7 g (20 mMol) Tetrabutylammoniumhydrogensulfat, 2,8 g (20 mMol) Methyljodid und 300 ml Benzol bestehendes Zweiphasensystem werden unter intensivem Rühren 2,84 g (10 mMol) 9,10 - Dihydrolysergsäuremethylester zugegeben. Es wird 30 Minuten gerührt und anschliessend werden die Phasen getrennt. Der wässerigen Phase werden 200 ml Benzol mit 2,1 g (15 mMol) Methyljodid zugegeben und 30 Minuten weitergerührt. Dann werden beide Phasen getrennt und die wässerige Phase noch 2 mal mit je 150 ml Benzol extrahiert. Alle vier Benzolextrakte werden mit Wasser bis zur neutralen Reaktion gewaschen und eingedampft. Es werden 2,7 g bzw. 91% d.Th. reinen, kristallinen 1 - Methyl - 9,10 - dihydrolysergsäuremethylesters erhalten. Die Verbindung hat die gleichen Eigenschaften wie jene im Beispiel 1.

Beispiel 3

2,7 g (10 mMol) 9,10-Dihydrolysergsäure und 6,44 g (20 mMol) Tetrabutylammoniumbromid werden in 200 ml 45%iger Natronlauge suspendiert. Es wird eine Lösung von 3,78 g (30 mMol) Dimethylsulfat in 300 ml Benzol zugegeben und 1 Stunde intensiv gerührt. Dann wird die organische Phase abgetrennt und die wässerige Phase in gleicher Weise noch 2 mal mit je 150 ml Benzol und 2,1 g (15 mMol) Methyljodid extrahiert. Die Benzolextrakte werden vereinigt, mit Wasser bis zur neutralen Reaktion gewaschen und im Vakuum eingedampft. Es werden 2,36 g bzw. 82,6% d. Th. reinen, kristallinen 1 - Methyl - 9,10 - dihydrolysergsäuremethylesters erhalten. Die Verbindung hat die gleichen Eigenschaften wie jene im Beispiel 1.

Beispiel 4

2,7 g (10 mMol) 9,10-Dihydrolysergsäure und 7 g (20 mMol) Tetrabutylammoniumhydrogensulfat werden in 200 ml 45%iger Natronlauge suspendiert und eine Lösung von 4,62 g (30 mMol) Diäthylsulfat in 300 ml Toluol wird zugegeben. Bei Raumtemperatur wird 8 Stunden intensive gerührt. Dann wird die organische Phase ab getrennt und die wässerige Phase in gleicher Weise noch 4 Stunden mit 300 ml Toluol und 1,54 g (10 mMol) Diäthylsulfat extrahiert. Die Toluolextrakte werden vereinigt, mit Wasser bis zur neutralen Reaktion gewaschen und im Vakuum eingedampft. Es werden 2,15 g bzw. 68,7% d. Th. reinen, kristallinen 1 - Äthyl - 9,10 - dihydrolysergsäureäthylesters mit einem Schmp. von 80—82°C und spezifischer Drehung $[\alpha]_D^{20}$ = −70,6° (c = 0,5; Chloroform) erhalten.

Beispiel 5

2,7 g (10 mMol) 9,10-Dihydrolysergsäure und 7 g (20 mMol) Tetrabutylammoniumhydrogensulfat werden in 200 ml 45%iger Natronlauge suspendiert, eine Lösung von 4,85 g (40 mMol) Allylbromid in 300 ml Benzol wird zugegeben und 2 Stunden intensiv gerührt. Dann werden die Phasen getrennt und die wässerige Phase in gleicher Weise noch 2 mal mit je 200 ml Benzol und 2,42 g (20 mMol) Allylbromid extrahiert. Die Benzolextrakte werden vereinigt, mit Wasser bis zur neutralen Reaktion gewaschen und im Vakuum eingedampft. Es werden 2,3 g bzw. 66% d. Th. 1-Allyl-9,10-dihydrolysergsäureallylester in der Form eines farblosen Harzes mit spezifischer Drehung von $[\alpha]_D^{20}$ = −69,9° (c = 0,5; Chloroform) erhalten.

Beispiel 6

0,3 g (1 mMol) 10-$\alpha$-Methoxy-lumilysergsäure und 0,7 g (2 mMol) Tetrabutylammoniumhydrogensulfat werden in 20 ml 45%iger Natronlauge suspendiert und diese Suspension wird 3 mal mit je 30 ml Benzol und 0,42 g (3 mMol) Methyljodid extrahiert, wobei jeweils eine Stunde intensiv gerührt wird. Die vereinigten Benzolextrakte werden mit Wasser gewaschen und im Vakuum eingedampft. Es werden 0,25 g bzw. 77% d. Th. 1-Methyl-10$\alpha$-methoxy - lumilysergsäuremethylester in der Form eines farblosen Harzes mit spezifischer Drehung $[\alpha]_D^{20}$ = +9° (c = 0,5; Chloroform) erhalten.

Beispiel 7

Ein Gemisch aus 4,5 g (13,22 mMol) 10$\alpha$-Methoxy-lumilysergsäuremethylester, 8,67 g (26,44 mMol) Tetrabutylammoniumhydrogensulfat, 200 ml 45%iger Natronlauge, 300 ml Toluol und 3,33 g (26,44 mMol) Dimethylsulfat wird 20 Minuten bei 30°C intensiv gerührt. Dann wird die Toluolphase von der Wasserphase getrennt und die Wasserphase noch 3 mal mit je 200 ml Toluol und 1,66 g (13,22 mMol) Dimethylsulfat extrahiert, wobei jeweils 20 Minuten intensiv bei 30°C gerührt wird. Die vereinigten Toluolextrakte werden mit Wasser gewaschen und im Vakuum eingedampft. Es werden 3,8 g 1-Methyl-10$\alpha$-methoxylumilysergsäuremethylester in der Form eines farblosen Harzes mit der spezifischen Drehung $[\alpha]_D^{20}$ = +8,7° (c = 0,5; Chloroform) erhalten.

Beispiel 8

In eine Suspension von 140 ml 45%iger Natronlauge, 4,87 g (14,3 mMol) Tetrabutylammoniumhydrogensulfat, 250 ml Benzol und 3 g (21,5 mMol) Methyljodid werden unter intensivem Rühren bei 35°C 2,6 g (7,16 mMol) 2-Brom-9,10-dihydrolysergsäuremethylester in 65 ml Benzol zugegeben. Die Lösung wird 30 Minuten gerührt und anschliessend wird die organische Phase von der wässerigen Phase getrennt. Die wässerige Phase wird noch 2 mal mit je 150 ml Benzol und 1,5 g (10,7 mMol) Methyljodid extrahiert. Die vereinigten Benzolextrakte werden mit Wasser gewaschen und im Vakuum eingedampft. Der Trockenrückstand wird aus Methanol um kristallisiert. Es werden 2,42 g bzw. 90% d. Th. kristallinen 1-Methyl-2-brom-9,10-dihydrolysergsäuremethylesters mit Schmp. 167—168°C und der spezifischen Drehung $[\alpha]_D^{20}$ = −94,2° (c = 0,5; Methanol) erhalten.

Beispiel 9

In eine Suspension aus 80 ml 45%iger Natronlauge, 3,5 g (10 mMol) Tetrabutylammoniumhydrogensulfat, 200 ml Cyclohexan und 3,51 g (22,5 mMol) Äthyljodid werden unter Rühren 1,45 g (5 mMol) 9,10-Dihydrolysergsäureäthylester zugegeben. Es wird 18 Stunden intensiv bei 60°C gerührt. Nach der Trennung beider Phasen wird die wässerige Phase noch einmal mit 150 ml Cyclohexan und 1,56 g (10 mMol) Äthyljodid extrahiert. Die Cyclohexanfraktionen werden vereinigt, mit Wasser gewaschen und eingedampft. Es werden 1,22 g bzw. 80% d. Th. 1-Äthyl-9,10-dihydrolysergsäureäthylester mit einem Schmp. von 80—83°C und einer spezifischen Drehung $[\alpha]_D^{20}$ = −70° (c = 0,5; Chloroform) erhalten.

Beispiel 10

In ein Zweiphasensystem, bestehend aus 200 ml 45%iger Natronlauge 7 g (20 mMol) Tetrabutylammoniumhydrogensulfat, 200 ml Toluol und 2,52 g (20 mMol) Dimethylsulfat, wird unter intensivem Rühren eine Lösung von 3,16 g (10 mMol) 9,10-Dihydrolysergsäure-2'-fluoräthylester in 100 ml Toluol zugegeben und 2 Stunden gerührt. Der Toluolextrakt wird von der wässerigen Phase getrennt und dann wird die wässerige Phase noch 2 mal mit je 200 ml Toluol und 0,63 g (5 mMol) Dimethylsulfat extrahiert. Die vereinigten Toluolextrakte werden mit Wasser gewaschen und im Vakuum bis zur Trockne eingedampft. Es werden 2,14 g

bzw. 71,8% d. Th. kristallinen 1-Methyl-9,10-dihydrolysergsäuremethylesters erhalten. Die Verbindung hat die im Beispiel 1 angegebenen Eigenschaften.

**Patentansprüche**

1. N-substituierte 9,10-Dihydrolysergsäureester der allgemeinen Formel I

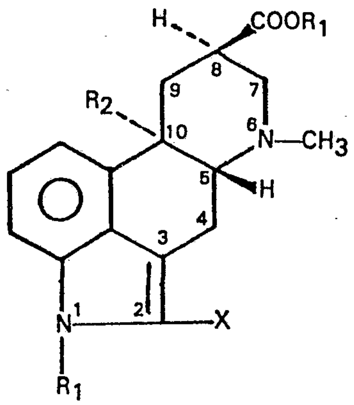

(I)

worin

$R_1$ einen Alkylrest mit 1—5 C-Atomen, einen Alkenylrest mit 2—5 C-Atomen oder einen Cycloalkylrest mit 3—5 C-Atomen,

$R_2$ ein Wasserstoffatom oder einen Alkoxyrest mit 1—3 C-Atomen und

X ein Wasserstoff-oder Halogenatom bedeuten, wobei die Verbindungen worin $R_1$ einen Methyl- oder Äthylrest und $R_2$ und X jeweils ein Wasserstoffatom bedeuten, sowie die Verbindungen, worin $R_1$ einen Methylrest, $R_2$ einen Methoxyrest und X ein Wasserstoffatom bedeuten, ausgenommen sind.

2. Verfahren zur Herstellung von N-substituierten 9,10-Dihydrolysergsäureestern der allgemeinen Formel I dadurch gekennzeichnet, dass der gegebenenfalls veresterte 9,10-Dihydrolysergsäure der allgemeinen Formel II

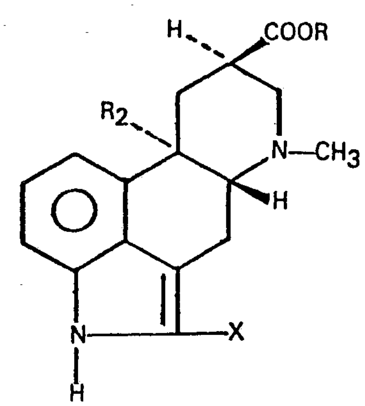

(II)

worin

R ein Wasserstoffatom oder einen in alkalischem Medium hydrolisierbaren organischen Rest bedeutet und

X und $R_2$ die angegebene Bedeutung haben, mit einer Verbindung der allgemeinen Formel III

$$R_1Y \quad (III)$$

worin

$R_1$ die angegebene Bedeutung hat und

Y das Äquivalent eines Halogen-oder Sulfations bedeutet,

in Anwesenheit eines Katalysators zur Phasenübertragung der allgemeinen Formel IV

$$[Z_4Q] \quad A \quad (IV)$$

in der

Z jeweils eine gleiche oder verschiedene Alkyl-, Cycloalkyl-, Aryl-, Arylalkyl- oder Alkylarylgruppe mit 1 bis 16 C-Atomen, Q ein quaternäres Stickstoff- oder ein Phosphoratom und A das Äquivalent eines Anions bedeutet, eines mit Wasser nicht mischbaren inerten organischen Lösungsmittels und einer alkalischen wässrigen Phase umgesetzt wird.

**Claims**

1. N-substituted 9,10-dihydrolysergic acid esters of the general formula I

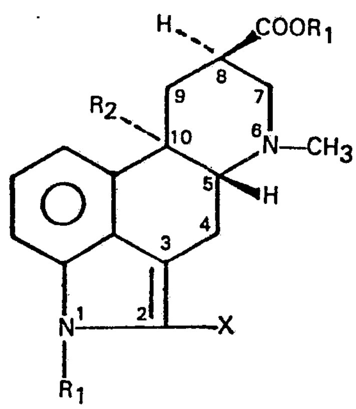

(I)

wherein

$R_1$ denotes an alkyl radical having 1—5 carbon atoms, an alkenyl radical having 2—5 carbon atoms or a cycloalkyl radical having 3—5 carbon atoms,

$R_2$ denotes a hydrogen atom or an alkoxy radical having 1—3 carbon atoms and

X denotes a hydrogen or a halogen atom, with the compounds being excepted, in which $R_1$ is a methyl or ethyl radical and $R_2$ and X respectively are a hydrogen atom, as well as compounds, in which $R_1$ is a methyl radical, $R_2$ is a methoxy radical and X is a hydrogen atom.

2. A process of preparing N-substituted 9,10-dihydrolysergic acid esters of the general formula I, characterized in that the optionally esterified 9,10-dihydrolysergic acid of the general formula II

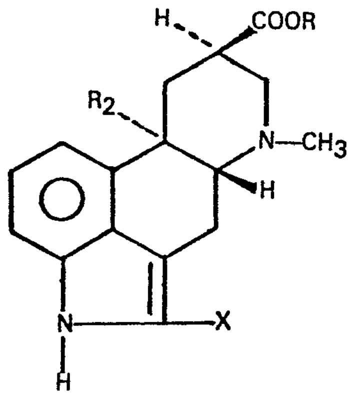

(II)

wherein

R denotes a hydrogen atom or an organic radical capable of being hydrolyzed in an alkaline medium and

X and $R_2$ have the stated meaning, is reacted with a compound of the general formula III

$$R_1Y \qquad (III)$$

wherein

$R_1$ has the stated meaning and

Y is the equivalent of a halogen or sulfate ion, in the presence of a catalyst for phase transfer of the general formula IV

$$Z_4Q \quad A \qquad (IV)$$

in which

Z respectively denotes a like or different alkyl, cycloalkyl, aryl, arylalkyl or alkylaryl group with 1 to 16 carbon atoms,

Q denotes a quaternary nitrogen atom or a phosphorus atom and

A denotes the equivalent of an anion, an inert organic solvent not miscible with water and an alkaline aqueous phase.

**Revendications**

1. Esters d'acides 9,10-dihydrolysergiques N-substitués de la formule générale I

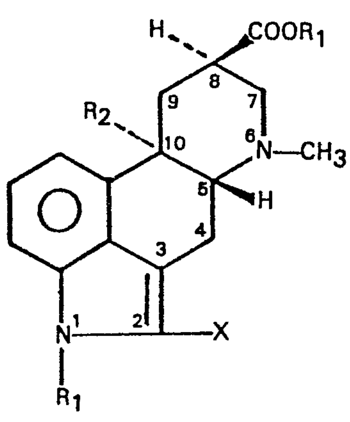

(I)

dans laquelle

$R_1$ est un reste d'alcoyle comprenant 1—5 atomes de C, un reste d'alcènyle comprenant 2—5 atomes de C ou un reste de cycloalcoyle comprenant 3—5 atomes de C,

$R_2$ un atome d'hydrogène ou un reste d'alcoxyle comprenant 1—3 atomes de C et

X un atome d'hydrogène ou d'halogène,

les composés dans lesquels $R_1$ est un reste de méthyle ou d'éthyle et $R_2$ et X sont respectivement un atome d'hydrogène, ainsi que les composés dans lesquels $R_1$ est un reste de méthyle, $R_2$ est un reste méthoxy et X est un atome d'hydrogène,

étant exceptés.

2. Procédé de préparation d'esters d'acides 9, 10-dihydrolysergiques N-substitués de la formule générale I, caractérisé en ce que l'acide 9,10-dihydrolysergique, éventuellement estérifié, de la formule générale II

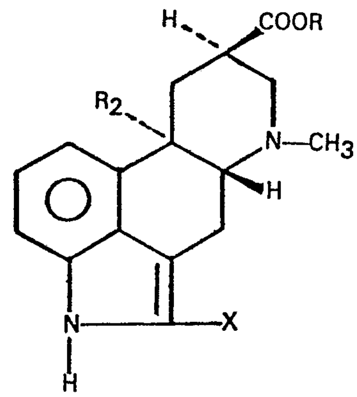

(II)

dans laquelle

R est un atome d'hydrogène ou un reste organique hydrolysable en milieu alcalin et

X et $R_2$ ont la signification indiquée,

et mis en réaction avec un composé de la formule générale III

$$R_1Y \qquad (III)$$

dans laquelle

$R_1$ a la signification indiquée et

Y est l'équivalent d'un ion d'halogène ou de sulfate,

en présence d'un catalysateur de transformation de phase de la formule générale IV

$$[Z_4Q] \quad A \qquad (IV)$$

dans laquelle

Z est constitué par, respectivement, les mêmes ou différents groupes d'alcoyle, cycloalcoyle, aryle, arylalcoyle ou d'alcoylaryle comprenant 1 à 16 atomes de C, Q est un atome quaternaire d'azote ou de phosphore et A est l'équivalent d'un anion, d'un solvant inerte organique non mélangeable avec de l'eau, et d'une phase alcaline aqueuse.